# EUROPEAN PATENT APPLICATION

(11) **EP 4 722 231 A1**
(43) Date of publication of application: **08.04.2026**
(21) Application number: 24838366.3
(22) Date of filing: 01.04.2024
(51) Int. Cl.: C07K 14/00, C12N 15/70, A61K 38/16, A61P 31/14

(54) **BIOSYNTHESIS METHOD FOR BROAD-SPECTRUM ANTIVIRAL POLYPEPTIDE**

(30) Priority: 11.07.2023 CN 202310848639; 27.11.2023 CN 202311595474
(71) Applicant: Shanxi Jinbo Bio-Pharmaceutical Co., Ltd., Shanxi 030032 (CN); Fudan University, Shanghai 200433 (CN)
(72) Inventor: LU, Lu, Shanghai 200433 (CN); JIANG, Shibo, Shanghai 200433 (CN); XIA, Shuai, Shanghai 200433 (CN); WANG, Liyu, Shanghai 200433 (CN); WANG, Qian, Shanghai 200433 (CN); YANG, Xia, Taiyuan, Shanxi 030032 (CN); HE, Zhenrui, Taiyuan, Shanxi 030032 (CN); ZHANG, Yongjian, Taiyuan, Shanxi 030032 (CN); LIU, Zengyao, Taiyuan, Shanxi 030032 (CN); WANG, Ying, Taiyuan, Shanxi 030032 (CN); LAN, Xiaobin, Taiyuan, Shanxi 030032 (CN); WANG, Lingling, Taiyuan, Shanxi 030032 (CN)
(74) Representative: Cabinet Becker et Associés
(86) International application number: PCT/CN2024/085311
(87) International publication number: WO 2025/011106

(57) **Abstract**

A biosynthesis method for a broad-spectrum antiviral polypeptide. The present invention specifically relates to a biosynthesis method for a polypeptide HCoV-EK1 capable of inhibiting human coronavirus infections in a broad-spectrum manner. The specific process comprises: construction of an Escherichia coli genetically engineered bacterium, fermentation culture of the Escherichia coli genetically engineered bacterium, and purification of recombinant polypeptide HCoV-EK1.

## Description

This application claims priority to the Chinese invention patent application No.: 202310848639.4 filed on July 11, 2023, and entitled "BIOSYNTHESIS METHOD FOR BROAD-SPECTRUM ANTIVIRAL POLYPEPTIDE", and the Chinese invention patent application No.: 202311595474.0 filed on November 27, 2023 and entitled "BIOSYNTHESIS METHOD FOR BROAD-SPECTRUM ANTIVIRAL POLYPEPTIDE".

### TECHNICAL FIELD

The present disclosure belongs to the field of synthetic biotechnology, and particularly relates to a polypeptide with broad-spectrum inhibitory activity against human coronavirus infection and a biosynthesis method thereof.

### BACKGROUND OF THE DISCLOSURE

The existence of human coronaviruses has brought a serious threat to human survival and health, and brought a serious impact on the global economic development. In addition, it has been found currently that some SARS-like viruses have gene sequences highly similar to that of SARS-CoV and also have the potential to infect humans. However, the existing SARS-CoV specific antibody drugs have no inhibitory effect on them. Therefore, the existence of such viruses also places human survival and health at a substantial potential risk.

At present, the research and development of inhibitors against such viruses are primarily directed to target two targets, namely the S1 region and the S2 region. Entry inhibitors acting on the S1 region are predominantly antibodies. While such antibodies exhibit favorable neutralizing activity against specific viruses, they fail to confer cross-protective efficacy against SARS-like viruses. Entry inhibitors targeting the S2 region are mainly polypeptide-based agents. However, there is still a lack of broad-spectrum design schemes for related inhibitors, with only designs based on the amino acid sequence or six-helix structure of the virus's native HR2 domain available. The human coronavirus polypeptide inhibitors reported to date therefore often lack broad-spectrum antiviral activity.

Patent CN 107022008 B uses a chemical synthesis method to prepare the HCoV-EK series of polypeptides, and researches have shown that the polypeptides within HCoV-EK series exhibit highly broad-spectrum and high-efficiency antiviral activity, and are capable of exerting favorable inhibitory effects against two or more types of human coronaviruses. The antiviral mechanism of such broad-spectrum entry-inhibitory polypeptides is primarily to interfere with the formation of the viral six-helix bundle by binding to the HR1 region of the viruses, thereby blocking the viral fusion and entry process.

At present, there are two main pathways to synthesize polypeptides: chemical synthesis and biosynthesis. The chemical synthesis is mainly achieved by the dehydration condensation reaction of amino acids. The chemical synthesis can be subdivided into solid-phase synthesis and liquid-phase synthesis depending on whether a solid-phase carrier resin is used. There are two strategies for liquid-phase synthesis, stepwise synthesis and fragment synthesis. Stepwise synthesis is simple and rapid and is suitable for most polypeptide synthesis, while fragment synthesis is suitable for polypeptides containing more than ten amino acids. Generally, the liquid-phase synthesis method has been replaced by the solid-phase synthesis method for the reasons such as its high pollution and complicated reaction processes. However, due to advantages such as large-scale production and low unit costs, the liquid-phase synthesis is still widely used in the synthesis of polypeptides with 10 or less amino acids. The principle of solid-phase polypeptide synthesis is to immobilize the C-terminus of amino acids on insoluble resin, and then carry out condensation reactions in sequence to extend the peptide chain. The solid-phase synthesis method can be subdivided into Fmoc method and Boc method. Fmoc method has become the first choice for the current polypeptide synthesis because of its advantages such as mild reaction conditions, simple reaction operation and ease of automation. However, there are still some overall problems in solid-phase polypeptide synthesis, such as relatively low reaction efficiency, difficulty in scaling up and high costs.

Biosynthesis mainly includes an enzymolysis method, an enzymatic catalysis method, a fermentation method and a genetic engineering method. The enzymolysis method refers to degrading macromolecular animal or plant proteins using biological enzymes to obtain small molecular peptides. However, this method is difficult for industrial production because of its low yield, increased pollution levels and unstable product quality. The fermentation method obtains polypeptides using microbial metabolism. Although the fermentation method has the advantage of low cost, it is difficult to obtain a specific polypeptide by fermentation of a specific microorganism. The enzyme catalysis method is especially suitable for the synthesis of polypeptide sequences due to its high activity and strong specificity. The enzyme catalysis method has the advantages such as being green and low industrialization cost. However, it also has the shortcomings such as difficulty in research and development and a longer research and development cycle. The genetic engineering method is based on DNA recombination technology, which controls the production of polypeptide sequences through DNA sequences. The genetic engineering method has the advantages such as strong site-specificity, low production costs, safety and environmental friendliness. However, it also faces the shortcomings such as large investment in research and development and production equipment, a longer research and development cycle, and the inability to express natural amino acids.

Based on the current state of the art, there is a need for new biosynthesis methods in the art.

### SUMMARY OF THE DISCLOSURE

The inventors have found that there is a technical problem that short polypeptides cannot be expressed or cannot be sufficiently expressed during the recombinant expression in *Escherichia coli* (*E. coli*), and therefore, for each polypeptide, it is necessary to conduct experiments to determine whether the polypeptide can be recombinantly expressed. It has been previously found that the polypeptide set forth in SEQ ID NO: 1 has antiviral activity. However, the polypeptide is prepared by chemical synthesis. No attempt to produce the polypeptide by microbial fermentation has been reported in the prior art. In this regard, the inventors have done extensive work to prepare the polypeptide shown in SEQ ID NO: 1 by microbial fermentation by selecting an appropriate coding sequence of SEQ ID NO: 2, and by using an appropriate fermentation and purification process.

In one aspect, the present disclosure provides a polynucleotide encoding the polypeptide set forth in SEQ ID NO: 1 comprising the nucleotide sequence set forth in SEQ ID NO: 2.

In one aspect, the present disclosure provides a vector comprising the polynucleotide described herein.

In one embodiment, the 5' end of the polynucleotide is linked (such as directly linked) to the nucleotide sequence of the TEV protease cleavage site to form an insertion cassette.

In one embodiment, the TEV protease cleavage site has the amino acid sequence of ENLYFQ.

In one embodiment, the vector comprises one or more of the followings: an N-terminal 6x His tag, a thrombin cleavage site, a C-terminal 6x His tag, a TrxA site, an enterokinase cleavage site, and an S tag.

In one embodiment, the vector is an expression vector. In one embodiment, the vector is a pET-32a (+) vector.

In one embodiment, the insertion cassette is inserted into the vector at the Kpn I and Xho I sites.

In one aspect, the present disclosure provides a host cell comprising the vector described herein. In one embodiment, the host cell is *E. coli.* In one embodiment, the *E. coli* is *E. coli* BL21 (DE3).

In one aspect, the present disclosure provides a composition comprising the polynucleotide, the vector, and the host cell described herein. In one embodiment, the composition is a kit.

In one aspect, the present disclosure provides a method of producing a polypeptide by fermentation, wherein the polypeptide is the polypeptide set forth in SEQ ID NO: 1, wherein the method comprises fermenting the host cell described herein under a condition suitable for culturing the host cell to collect the polypeptide in a supernatant or the cell.

In one aspect, the present disclosure provides a method of producing the polypeptide of SEQ ID NO: 1 by fermentation, comprising fermenting a host cell under a condition suitable for culturing the host cell to collect the polypeptide in a culture supernatant or the host cell, wherein the host cell is a prokaryote host cell and comprises the polynucleotide of SEQ ID NO: 2.

In one embodiment, the host cell is an *E. coli* cell, and the method comprises one or more of the following steps:
(1) culturing the *E. coli* cell in LB medium to obtain a seed culture;
(2) scaling up the seed culture for an appropriate time, and performing an induction culture;
(3) collecting and lysing the *E. coli* cell, and separating the lysate to obtain a supernatant and a pellet;
(4) loading the supernatant into a column to separate and collect the polypeptide; and
(5) enzymatically digesting the polypeptide to remove the tag linked to the polypeptide.

In one embodiment, the step (1) comprises culturing the *E. coli* cell in LB medium at 35-38°C, preferably at 37°C for 6-20 hours, preferably 8-16 hours to obtain the seed culture.

In one embodiment, the step (2) comprises adding the seed culture to an expansion medium, preferably LB medium, in a ratio of the expansion medium to the seed culture of 10:1 to 200:1, preferably 50:1 to 150:1, more preferably 80 to 120:1 followed by incubation at 35-38°C, preferably 37°C for 6-10 hours, preferably 7-8 hours; conducting induction by adding IPTG at a IPTG concentration of 0.2-1 mM, preferably 0.3-8 mM, preferably 0.5 mM, cooling to 10-20°C, preferably 12-18°C, preferably 16°C, conducting induction culture for 6-20 hours, preferably 8-16 hours, and collecting the cell.

In one embodiment, the step (3) comprises resuspending the cell in a buffer solution, cooling to ≤ 15 °C, performing a homogenization process, preferably a high-pressure homogenization process, to obtain a homogenate, and then separating the homogenate into the supernatant and the pellet, for example by centrifugation.

In one embodiment, the step (4) comprises equilibrating column material with an equilibration solution, loading the supernatant into the column material, washing out impurities with a washing solution to remove impurities, and eluting the column with an eluting solution to obtain the polypeptide. Preferably, the column material is a nickel column.

In one embodiment, the step (5) comprises incubating the polypeptide with a TEV protease, preferably a TEV protease having a His tag, for example at 10-20 °C for 1-8 hours, such as at 16 °C for 2 hours, to obtain the polypeptide.

In one embodiment, the equilibration solution and/or the washing solution comprises 100-400 mM, such as 200-300 mM sodium chloride, 10-50 mM, such as 20-40 mM Tris and 10-50 mM, such as 20-40 mM imidazole; the eluting solution comprises 100-400 mM, such as 200-300 mM sodium chloride, 10-50 mM, such as 20-40 mM Tris and 100-500 mM, such as 200-400 mM imidazole.

In one embodiment, the method further comprises the step of subjecting the culture supernatant or lysate comprising the polypeptide or an eluate comprising the polypeptide to a reversed-phase column chromatography.

In one embodiment, the reversed-phase column is packed with a material having the following properties:
(1) the shape of the silica gel particles is spherical; (2) the pore size is 100-1000 angstroms; (3) the particle size is 3-15 µm; and (4) the bonded phase is ODS, C1, C4, C8, or APS.

In one embodiment, the reversed-phase column is a Daisogel SP-100-8-c8-PK type reversed-phase column.

In another aspect, the present disclosure provides a biosynthesis method for a polypeptide TEKB inhibiting a human coronavirus infection, comprising: (1) constructing a genetically engineered *E. coli* cell; (2) fermenting and culturing the genetically engineered *E. coli* cell; (3) purifying the recombinant polypeptide TEKB.

In another aspect, the present disclosure provides a method of inhibiting a coronavirus comprising applying a polypeptide of SEQ ID NO: 1 or a composition comprising the polypeptide, wherein the polypeptide has no acetyl group at the N-terminal amino acid residue and/or has no amide group at the C-terminal amino acid residue or is not acetylated at the N-terminal amino acid residue and/or not amidated at the C-terminal amino acid residue; or the polypeptide is a polypeptide expressed by a prokaryote host cell or is not a chemically synthesized polypeptide, wherein the coronavirus is selected from the group consisting of SARS-CoV-2, Rs3367-CoV, WIV1-CoV, MERS-CoV and HCoV-229E. In one embodiment, SARS-CoV-2 is selected from the group consisting of BA.2, BA.2.75, BA.2.86, XBB.1.5, and EG.5 variants. In one embodiment, the polypeptide or the composition comprising the polypeptide is administered to a cell infected with or at risk of being infected with a coronavirus or is contacted with a coronavirus. In one embodiment, the polypeptide is the polypeptide prepared by the method described herein. In one embodiment, the composition further comprises an agent for inhibiting coronavirus or for treating and/or preventing a disease caused by the coronavirus, preferably selected from the group consisting of favipiravir, nelfinavir, arbidol, lopinavir, ritonavir, chloroquine phosphate, darunavir, or remdesivir. In one embodiment, the composition comprises a pharmaceutically acceptable carrier. In one embodiment, the method is an *in vitro* method.

In another aspect, the present disclosure provides use of the polypeptide of SEQ ID NO: 1 or a composition comprising the polypeptide in the manufacture of a medicament or a kit for preventing or treating a coronavirus infection or a disease caused by a coronavirus in a subject, wherein the polypeptide has no acetyl group at the N-terminal amino acid residue and/or has no amide group at the C-terminal amino acid residue or has no acetylation at the N-terminal amino acid residue and/or has no amidation at the C-terminal amino acid residue; or the polypeptide is a polypeptide expressed by a prokaryote host cell or is not a chemically synthesized polypeptide. Also provided are a method for preventing or treating a coronavirus infection or a disease caused by a coronavirus in a subject, comprising administering to the subject the polypeptide described herein. The coronavirus may be selected from the group consisting of SARS-CoV-2, Rs3367-CoV, WIV1-CoV, MERS-CoV and HCoV-229E. In one embodiment, SARS-CoV-2 is selected from the group consisting of BA.2, BA.2.75, BA.2.86, XBB.1.5, and EG.5 variants. In one embodiment, the polypeptide is the polypeptide prepared by the method described herein. In the present disclosure, the polypeptide is a polypeptide expressed in a prokaryotic cell, such as *E. coli,* and no additional amino acid sequence is comprised after expression or purification. In one embodiment, the dosage form of the medicament is a tablet, a capsule, a dropping pill, an aerosol, a pill, a powder, a solution, a suspension, an emulsion, a granule, a liposome, a transdermal agent, a suppository or a lyophilized powder injection. In one embodiment, the composition further comprises an agent for inhibiting coronavirus or for treating and/or preventing a disease caused by coronavirus, preferably selected from the group consisting of favipiravir, nelfinavir, arbidol, lopinavir, ritonavir, chloroquine phosphate, darunavir, or remdesivir. In one embodiment, the composition comprises a pharmaceutically acceptable carrier. In one embodiment, the medicament is preferably administered by injection, including subcutaneous injection, intravenous injection, intramuscular injection and intraperitoneal injection, intracisternal injection or infusion, and the like, cavity administration, such as rectal, vaginal and sublingual, respiratory administration, such as nasal; mucosal administration or topical administration.

Advantages of the present disclosure include:
1. In the present disclosure, the recombinant polypeptide HCoV-EK1 (TEKB) is prepared using the novel biosynthesis method by fermentation in *E. coli.* The polypeptide has a very broad-spectrum and high-efficiency antiviral activity, and is capable of providing an excellent inhibitory effect on two or more human coronaviruses. The method has the advantages of having a simple production process, a low cost and ease of popularization.
2. The method of the present disclosure has a high polypeptide yield and/or recovery. The concentration of the purified polypeptide TEKB was 7.90 mg/ml, the peptide yield in 2 L fermentation broth is 158 mg, and the protein recovery rate relative to the fermentation broth is as high as 79 mg/L.
3. Through comparison, it is found that the preparation of the recombinant polypeptide HCoV-EK1 (TEKB) by the biosynthesis method has the advantages of having a simple production process, a low cost, a high production, no organic solvent addition, and no change in amino acid sequence and the like.
4. The inventors have discovered an optimal reversed-phase column packing material (such as a reversed-phase Daisogel SP-100-8-c8-PK (Daisogel) column) suitable for purification of the polypeptide TEKB at a high purity level. Compared with other separation columns, the reversed-phase Daisogel SP-100-8-c8-PK (Daisogel) column can separate and obtain the polypeptides of the present disclosure with a higher purity.
5. Although the antiviral activity of conventional chemically synthesized polypeptide fusion inhibitors is usually significantly superior to that of polypeptides expressed in prokaryote cells (Biochemical and Biophysical Research Communications 319 (2004) 283-288), in the present disclosure, the inventors have found that compared with the chemically synthesized EK1 polypeptide, the EK1 polypeptide expressed in a prokaryotic cell has an equivalent or even higher viral inhibitory activity, which is an important inventive feature of the present disclosure.

### DESCRIPTION OF THE DRAWINGS

Fig. 1 shows the electrophoretic detection result of the polypeptide TEKB.
Fig. 2 shows the purity detection result of the polypeptide TEKB obtained using different purification columns.
Fig. 3 shows the mass spectrometry identification result of the polypeptide TEKB.
Fig. 4 shows a vector map of pET-32a-TEKB for expressing the polypeptide.
Fig. 5 shows the inhibitory activity of the prokaryotic cell-expressed polypeptide reEK1 against SARS-CoV-2 (D614G) pseudovirus and its Omicron variant (BA.2) pseudovirus.
Fig. 6 shows the inhibitory activity of the prokaryotic cell-expressed polypeptide reEK1 against BA.2.75 and XBB.1.5 pseudoviruses.
Fig. 7 shows the inhibitory activity of the prokaryotic cell-expressed polypeptide reEK1 against XBB.1.5-mediated membrane fusion and XBB.1.5 pseudovirus.
Fig. 8 shows the inhibitory activity of the prokaryotic cell-expressed polypeptide reEK1 against BA.2.86-mediated membrane fusion and BA.2.86 pseudovirus.
Fig. 9 shows the antiviral effect of the prokaryotic cell-expressed polypeptide reEK1 on Omicron variant (BA.2) *in vivo.*

### DETAILED DESCRIPTION

The present disclosure is further illustrated by the following embodiments, but any embodiments or combinations thereof should not be construed as limiting the scope or embodiments of the present disclosure. The scope of the present disclosure is defined by the accompanying claims, and the scope defined by the claims will be apparent to those of ordinary skill in the art in combination with the specification and general knowledge in the art. Without departing from the spirit and scope of the present disclosure, those skilled in the art can make any modifications or changes to the technical solutions of the present disclosure, and such modifications and changes are also included in the scope of the present disclosure.

The inventors have found that there is a technical problem that short polypeptides cannot be expressed or cannot be sufficiently expressed during the recombinant expression of *E. coli,* and therefore, for each polypeptide, it is necessary to conduct experiments to determine whether the polypeptide can be recombinantly expressed. It has been previously found that the polypeptide set forth in SEQ ID NO: 1 has antiviral activity. However, the polypeptide is prepared by chemical synthesis. No attempt to produce the polypeptide by microbial fermentation has been reported in the prior art. In this regard, the inventors have done extensive work to prepare the polypeptide shown in SEQ ID NO: 1 by microbial fermentation, by selecting an appropriate coding sequence of SEQ ID NO: 2 and using an appropriate fermentation and purification process. The methods of the present disclosure can recombinantly produce the polypeptide set forth in SEQ ID NO: 1 at a high yield. After calculation, the concentration of the purified polypeptide TEKB is 7.90 mg/ml, the peptide yield from 2L fermentation broth is 158 mg, and the protein recovery relative to the fermentation broth is as high as 79 mg/L, with the purity up to 99.7%.

As used herein, a peptide or polypeptide refers to a chain of a plurality of amino acid residues linked by peptide bonds. The polypeptide may be a polypeptide set forth in SEQ ID NO: 1, which may be labeled as a TEKB polypeptide. The present disclosure finds that a large quantity of the polypeptide shown in SEQ ID NO: 1 can be present in cells in a soluble form, which is suitable for isolation and purification. Herein, the coding sequence of the polypeptide is linked to the coding sequence of a TEV protease cleavage site to facilitate subsequent enzymatic cleavage and purification. The polypeptide set forth in SEQ ID NO: 1 herein is a polypeptide naturally synthesized by prokaryotic cells, such as *E. coli.*

As used herein, a "nucleic acid" refers to a plurality of nucleotides linked by internucleotide linkages. The internucleotide linkage may be, for example, a phosphodiester bond. The nucleic acid herein may comprise a polynucleotide encoding a polypeptide of the present disclosure. To facilitate subsequent processing of the polypeptide, the nucleic acid of the present disclosure may also comprise polynucleotide encoding a purification tag, such as a His tag, a GST tag, an MBP tag, a SUMO tag or a NusA tag, and, if desired, a nucleotide sequence encoding a leader sequence. The polynucleotides of the present disclosure may be codon-optimized for host cells expressing the polypeptide, such as codon-optimized for *E. coli* expression. Herein, the nucleic acid or polynucleotide may be or comprise the nucleotide sequence set forth in SEQ ID NO: 2. The polynucleotide can produce the polypeptide of interest in high yield in *E. coli.* The 5' end of the polynucleotide can be linked to the nucleotide sequence encoding the TEV protease cleavage site to form an insertion cassette. The TEV protease cleavage site has the amino acid sequence of ENLYFQ.

As used herein, the term "vector" is a nucleic acid delivery vehicle into which a polynucleotide can be inserted. When the vector enables expression of the protein encoded by the inserted polynucleotide, the vector is referred to as an expression vector. The vector can be introduced into a host cell by transformation, transduction or transfection, so that the genetic material elements carried by the vector can be expressed in the host cell. Vectors are well known to those skilled in the art and include, but are not limited to: a plasmid; a phagemid; a cosmid; an artificial chromosome, such as a yeast artificial chromosome (YAC), a bacterial artificial chromosome (BAC), or P1-derived artificial chromosome (PAC); a bacteriophage such as λ phage or M13 phage and animal viruses. The vector may contain a variety of elements that control expression, including, but not limited to, a promoter sequence, a transcription initiation site, an enhancer sequence, a selection element, and a reporter gene. Additionally, the vector may also contain a replication initiation site. The vector may comprise a nucleic acid of the present disclosure to facilitate introduction into a cell for expression. The vector may comprise expression control elements, such as a promoter, a terminator, and/or an enhancer, operably linked to the nucleic acid. Herein, the vector may comprise one or more of an N-terminal 6x His tag, a thrombin cleavage site, a C-terminal 6x His tag, a TrxA site, an enterokinase cleavage site, and an S tag. The vector may be a pET-32a (+) vector.

The term "host cell" is a cell into which a nucleic acid molecule has been introduced by molecular biological techniques. These techniques include transfection of a viral vector, transformation with a plasmid vector, and introduction of naked DNA by electroporation, lipofection, and particle gun bombardment. The host cell may be a eukaryotic cell or a prokaryotic cell. For example, the eukaryotic cell can be a yeast cell, an animal cell, and/or an insect cell. The prokaryotic cell may be an *E. coli* cell. Particularly preferred herein is an *E. coli* cell, in particular *E. coli* BL21 (DE3).

### Construction of a host cell

Construction of the host cell of the present disclosure comprises cloning the nucleotide sequence encoding the polypeptide TEKB into an expression vector, and then transferring the expression vector (e.g., by transformation or transduction) into a host cell (e.g., *E. coli*) expression strain, and screening for genetically engineered bacteria (e.g., engineered *E. coli).* The host cell may be a variety of cells, such as a eukaryotic cell or a prokaryotic cell. The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Staphylococcus, Streptococcus,* and *Streptomyces.* Gram-negative bacteria include, but are not limited to, *Campylobacter, E. coli, Flavobacterium, Fusobacterium, Helicobacter, Neisseria, Pseudomonas, Salmonella,* and *Ureaplasma.* The host cell may also be a eukaryote, such as a mammalian, insect, plant or fungal cell. In this disclosure, a particularly preferred host cell is an *E. coli* cell, in particular *E. coli* BL21 (DE3).

Herein, the nucleotide sequence encoding the polypeptide TEKB is a nucleotide sequence that is codon-optimized for a host cell, in particular a nucleotide sequence that is codon-optimized for *E. coli.* The nucleotide sequence may be:

AGCCTGGATCAGATTAATGTGACCTTTCTGGATCTGGAATATGAAATGAAAA AGCTGGAAGAAGCCATTAAGAAACTGGAAGAAAGTTATATTGACCTGAAAGAACTG (SEQ ID NO.2). The inventors find that this sequence is particularly suitable for *E. coli* expression of the polypeptide TEKB described herein.

The nucleotide sequence of the polypeptide TEKB can be cloned into an expression vector by a variety of conventional methods. The type of the expression vector is not particularly limited, and includes, for example, pET-32a, pET-28a, and the like. A particularly preferred expression vector herein is the pET-32a (+) vector. The inventors have found that this expression vector is particularly suitable for expressing the nucleotide sequences or polypeptides described herein. For example, the pET-32a (+) vector may comprise a TrxA tag, a His tag, a thrombin site, and an S tag. These tags can be expressed together with the TEKB polypeptide. The N-terminus of the coding sequence of the polypeptide TEKB can be linked to the coding sequence of the TEV protease cleavage site to facilitate subsequent enzymatic cleavage of these tags and purification. The genetically engineered bacteria (e.g., genetically engineered *E. coli*) can be screened by transferring the expression vector into a host cell, such as a strain of *E. coli,* by techniques known in the art, such as transformation or transduction. The inventors find that the polypeptide TEKB can be expressed in high yield and/or obtained in high recovery and/or high purity in any of the following cases: expression with the nucleotide of SEQ ID NO. 2, elution of a column material (such as a nickel column such as Ni6FF) with the eluting solution described herein, and enzymatic digestion by TEV protease. From extensive experiments, the inventors find that conventional expression vectors have problems such as low yield and/or low recovery of the expression product, and the expression product is difficult to be efficiently cleaved by enzymes. The present disclosure can produce the polypeptide of interest in high yield, high recovery, and high purity. The initially expressed expression product can be efficiently cleaved by TEV protease.

### Fermentation culture and production of the host cell

Herein, the host cell is an *E. coli* cell, and the method of producing a polypeptide may comprise one or more of the following steps:
(1) culturing the *E. coli* cell in a medium (such as LB medium) to obtain a seed culture;
(2) scaling up the seed culture for an appropriate time, and performing an induction culture;
(3) collecting and lysing the *E. coli* cell, and separating the lysate to obtain a supernatant and a pellet;
(4) loading the supernatant into a column to separate and collect the polypeptide; and
(5) enzymatically digesting the target polypeptide to remove a tag linked to the polypeptide.

The step (1) may comprise culturing the *E. coli* cell in a culture medium at 35-38°C, preferably 37°C for 6-20 hours, preferably 8-16 hours to obtain a seed culture. The incubation temperature may be 35, 36, 37 or 38 °C. The incubation time may be, for example, 7, 8, 9, 10, 11, 12, 13, 14, 15, 16, 17, 18 or 19 hours.

The step (2) may comprise adding the seed culture to an expansion medium in a ratio of the expansion medium to the seed culture of 10: 1 to 200: 1, preferably 50: 1 to 150: 1, more preferably 80 to 120: 1. The ratio of the expansion medium to the seed culture may be 20:1, 30:1, 40:1, 50:1, 60:1, 70:1, 80:1, 90:1, 100:1, 110:1, 120:1, 130:1, 140:1, 150:1, 160:1, 170:1, 180:1, or 190:1. The expansion medium may be a LB medium. The culture conditions in the expansion medium are 35-38 °C (such as 35, 36, 37 or 38 °C), preferably 37 °C for 6-10 hours, such as 7, 7.5, 8, 8.5, 9 or 9.5 hours. After culture, an inducing agent (such as IPTG) can be added to induce expression. The concentration of the inducing agent (such as IPTG) is 0.2-1 mM, preferably 0.3-0.8 mM, preferably 0.5 mM. For example, the concentration of the inducing agent may be 0.3, 0.4, 0.5, 0.6, 0.7, 0.8, or 0.9 mM.

After addition of the inducing agent, the culture can be cooled down to 10-20 °C, for example 11, 12, 13, 14, 15, 16, 17, 18 or 19 °C, preferably 16 °C for induce culture for 6-20 hours, such as 7, 8, 9, 10, 11, 12, 13, 14, 15, 16, 17, 18 or 19 hours. Then, the bacteria can be collected. The collection for bacteria can be carried out by various means, for example by centrifugation.

The step (3) may comprise resuspending the bacteria in a buffer solution. The buffer solution may be an equilibration solution for subsequent purification processes. For example, the buffer solution may comprise 100-400 mM, such as 200-300 mM sodium chloride, 10-50 mM, such as 20-40 mM Tris, and 10-50 mM, such as 20-40 mM imidazole. The sodium chloride concentration of the buffer solution may be 110, 120, 130, 140, 150, 160, 170, 180, 190, 200, 210, 220, 230, 240, 250, 260, 270, 280, 290, 300, 310, 320, 330, 340, 350, 360, 370, 380, or 390 mM. The concentration of Tris may be 10, 15, 20, 25, 30, 35, 40, 45, or 50 mM. The concentration of imidazole may be 10, 15, 20, 25, 30, 35, 40, 45, or 50 mM. The suspension may be cooled to ≤ 15 °C, for example 1, 2, 3, 4, 5, 6, 7, 8, 9, 10, 11, 12, 13, 14 or 15 °C for homogenization to obtain a homogenate. The homogenization treatment may be a high-pressure homogenization treatment. The homogenization treatment may be performed one or more times, for example 2-8 times, such as 2, 3, 4, 5, 6 or 7 times. The homogenate may be separated into a supernatant and a pellet, for example by centrifugation.

The step (4) may comprises equilibrating the column material with an equilibration solution, loading the supernatant to the column material, washing the column material with a washing solution to remove the impurities, and eluting with an eluting solution to obtain the polypeptide. The equilibration solution and/or the washing solution comprises 100-400 mM, such as 200-300 mM sodium chloride, 10-50 mM, such as 20-40 mM Tris, and 10-50 mM, such as 20-40 mM imidazole. The concentration of sodium chloride may be 110, 120, 130, 140, 150, 160, 170, 180, 190, 200, 210, 220, 230, 240, 250, 260, 270, 280, 290, 300, 310, 320, 330, 340, 350, 360, 370, 380, or 390 mM. The concentration of Tris may be 10, 15, 20, 25, 30, 35, 40, 45, or 50 mM. The concentration of imidazole may be 10, 15, 20, 25, 30, 35, 40, 45, or 50 mM.

The eluting solution may comprise 100-400 mM, such as 200-300 mM sodium chloride, 10-50 mM, such as 20-40 mM Tris, and 100-500 mM, such as 200-400 mM imidazole. The concentration of sodium chloride may be 110, 120, 130, 140, 150, 160, 170, 180, 190, 200, 210, 220, 230, 240, 250, 260, 270, 280, 290, 300, 310, 320, 330, 340, 350, 360, 370, 380, or 390 mM. The concentration of Tris may be 10, 15, 20, 25, 30, 35, 40, 45, or 50 mM. The concentration of imidazole may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 mM.

The step (5) may comprise incubating the polypeptide with a TEV protease or a TEV protease having a His tag. For example, the polypeptide is incubated at 10-20 °C, such as 11, 12, 13, 14, 15, 16, 17, 18, 19 or 20 °C for 1-10 hours, such as 1, 2, 3, 4, 5, 6, 7, 8 or 9 hours to obtain a polypeptide.

The method of the present disclosure may further comprise the step of subjecting the eluate comprising the polypeptide to reversed-phase column chromatography.

The method of the present disclosure may further comprise the step of subjecting the supernatant or lysate (i.e., the supernatant obtained by centrifugation after cell lysis) comprising the polypeptide to reversed-phase column chromatography.

The step of reversed-phase column chromatography is performed.

The reversed-phase column chromatography may use a packing material having the following properties: (1) the shape of the silica gel particles is spherical; (2) the pore size is 100-1000 angstroms; (3) the particle size is 3-15 µm; and (4) the bonded phase is ODS, C1, C4, C8, or APS.

The packing material for reversed-phase column chromatography may be spherical fully porous silica gel. The bonded phase of the packing material in reversed-phase column chromatography may include C18 (ODS), C8, C4, APS (amino), phenyl, cyano, diol, and the like. The packing material may have various pore sizes, preferably 60-2000 Angstroms, such as 100, 110, 120, 130, 140, 150, 160, 170, 180, 190, 200, 210, 220, 230, 240, 250, 260, 270, 280, 290, 300, 400, 500, 600, 700, 800, 900, 1000, 1100, 1200, 1300, 1400, 1500, 1600, 1700, 1800, 1900, or 2000 Angstroms. The packing material for the reversed-phase column chromatography may have various particle sizes, for example a particle size of 1-50 µm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50µm. For example, for a pore size of 60 Angstroms, it may have a particle size of 3-5 µm, such as a particle size of 3, 4 or 5 µm. For a pore size of 100 Angstroms, it may have a particle size of 1-20 µm, such as a particle size of 1.7, 2.1, 2.5, 3, 5, 10, or 15µm. For a pore size of 120 Angstroms, it may have a particle size of 1-60 µm, such as a particle size of 1.7, 2.1, 2.5, 3, 5, 10, 15, 20, 40, or 50 µm. For a pore size of 200 Angstroms, it may have a particle size of 10-15 µm. For a pore size of 300 Angstroms, it may have a particle size of 10-60 µm, such as 10, 20, 30, 40 or 50µm. For a pore size of 1000 Angstroms, it may have a particle size of 1-10 µm, such as a particle size of 1, 2, 3, 4, 5, 6, 7, 8, 9, or 10µm. For a pore size of 2000 Angstroms, it may have a particle size of 10-60 µm, such as 10, 20, 30, 40 or 50µm.

The inventors have found in a long-term work that the high purity purification of the polypeptide can be achieved by using a packing material that meets the following criteria:: (1) the shape of the silica gel particles is spherical; (2) the pore size is 100-1000 angstroms; (3) the particle size is 3-15 µm; and (4) the bonded phase is ODS, C1, C4, C8 or APS, particularly Daisogel SP-100-8-c8-PK type packing material. The high purity may be 80%-100% purity, such as 90%, 91%, 92%, 93%, 94%, 95%, 96%, 97%, 98%, 98.5%, 99.0%, 99.1%, 99.2%, 99.3%, 99.4%, 99.5%, 99.6%, 99.7%, 99.8%, 99.9%, or 100% purity.

The purification conditions of the polypeptides described herein may be:
Mobile phase: 0.1% TFA aqueous solution and acetonitrile (90% and 10%) for 0-60 min;
0.1% TFA aqueous solution and acetonitrile (45% and 55%) at 60 min; detection wavelength: 215 nm 270 nm; volumetric flow rate: 3 ml/min.

### Pseudoviruses Preparation

Pseudoviruses can be prepared herein using methods known in the art. For example, as previously described (L. Lu, Q. Liu, Y. Zhu, K.-H. Chan, L. Qin, Y. Li, Q. Wang, J. F.-W. Chan, L. Du, F. Yu, C. Ma, S. Ye, K.-Y. Yuen, R. Zhang, S. Jiang, Structure-based discovery of Middle East respiratory syndrome coronavirus fusion inhibitor. Nat. Commun. 5, 3067 (2014), or Xing L., et al. A five-helix-based SARS-CoV-2 fusion inhibitor targeting Heptad Repeat 2 domain against SARS-CoV-2 and its variants of concern. Viruses. 2022 Mar 13;14(3):597), pseudoviruses can be obtained by transfecting 293T cells with plasmids, including HIV backbone plasmid pNL4-3.Luc.R⁻.E⁻ and a plasmid for expressing the S protein of different SARS-CoV-2 variants. The amino acid sequences of the S proteins of different SARS-CoV-2 variants are known.

### Comparison of advantages and disadvantages of chemical synthesis and biosynthesis preparation processes of the polypeptide TEKB

**Table 1**

| | Biosynthesis method | Chemical synthesis method |
|---|---|---|
| Production cycle | 5-8 days | 10-14 days |
| Production costs | 23.5 yuan/1 mg polypeptide | 145.1 yuan/1 mg polypeptide |
| Preparation Process | The process is simple to operate, and no harmful components such as organic solvents are used. | The process is complicated, requires repeated purification, and uses a large amount of organic solvent. |
| yield | 79 mg/L fermentation broth | The amount prepared in a time is 10 mg. |
| Influence on amino acid sequence | not alter the amino acid sequence of the polypeptide. | N- or C-terminal amino acid is typically protected by acetylation or amidation. |

Through comparison, it is found that the preparation of the recombinant polypeptide HCoV-EK1 (TEKB) by the biosynthesis method has the advantages such as simple production processes, a low cost, a high yield, no organic solvent addition, and no change on the amino acid sequence and the like.

The sequence of EK1 is Ac-SLDQINVTFLDLEYEMKKLEEAIKKLEESYIDLKEL-NH2 (Ac is acetyl, NH2 is amidated). The TEKB (EK1) expressed by prokaryotic cells of the present disclosure is referred to as reEK1 (the polypeptide expressed by *E. coli,* contains no modification of N- or C-terminal amino acid residues; the amino acid sequence is SLDQINVTFLDLEYEMKKLEEAIKKLEESYIDLKEL); the chemically synthesized EK1 in the present application is referred to as EK1.

### Examples

The following examples are provided for illustrating the present disclosure. It should be understood by those skilled in the art that the embodiments are merely illustrative and not limiting. The present disclosure is limited only by the scope of the accompanying claims.

### Example 1: Production of Polypeptide TEKB

### (1) Construction of genetically engineered E. coli

1. The amino acid sequence corresponding to the polypeptide TEKB is: SLDQINVTFLDLEYEMKKLEEAIKKLEESYIDLKEL (SEQ ID NO.1).
2. The nucleotide sequence for synthesizing the corresponding amino acid sequence is: AGCCTGGATCAGATTAATGTGACCTTTCTGGATCTGGAATATGAAATGAAAAAGCTGGA AGAAGCCATTAAGAAACTGGAAGAAAGTTATATTGACCTGAAAGAACTG (SEQ ID NO.2).
3. The nucleotide sequence encoding the polypeptide TEKB was cloned into the expression vector pET-32a (+), after which the expression vector was transferred into the *E. coli* expression strain, and screening was performed to obtain the genetically engineered *E. coli.*

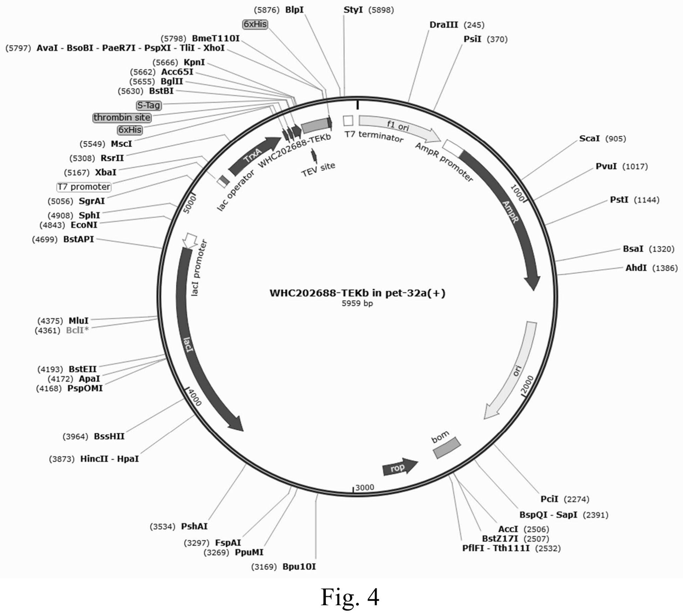

Specifically, according to the amino acid sequence of TEKB, codons preferred by *E. coli* were optimized and selected to generate the corresponding gene sequence, namely SEQ ID NO.2. BEIJING LIUHE CO., LIMITED was commissioned to ligate the TEKB gene fragment (SEQ ID NO. 2) to the gene sequence encoding the TEV protease cleavage site: the gene sequence corresponding to ENLYFQ was ligated to the 5' end of the TEKB gene. The resulting fused sequence was then inserted into the pET-32a expression vector (BEIJING LIUHE CO., LIMITED) via the restriction enzyme sites of Kpn I (NEB Inc., Cat.: R0136L) and Xho I (NEB Inc., Cat.: R0146L) to construct the pET-32a-TEKB expression vector (see Fig. 4). The expression vector was introduced into *E. coli* BL21 (DE3), and positive genetically engineered *E. coli* strains were screened out.

### (2) Fermentation culture of genetically engineered E. coli

a. A preferred single colony of genetically engineered *E. coli* was picked out and placed in 5 mL of LB culture medium for overnight culture at 35-38 °C, preferably at 37 °C.
b. The bacterial broth was inoculated at a ratio of 1:100 for scale-up culture (2 L fermentation broth), cultured at 37 °C for 7 hours, and then 0.5 mM IPTG was added for induction. The culture was cooled to 16 °C and subjected to induction culture overnight. The protein expressed at this stage was TEKB. The bacteria were collected by centrifugation.
c. The collected bacteria were resuspended with equilibration working solution (200 mM sodium chloride, 25 mM Tris, 20 mM imidazole), and the bacterial suspension was cooled to ≤ 15°C prior to homogenization. High-pressure homogenization was performed twice (the homogenate obtained from the first homogenization was labeled as homogenate #1; the homogenate obtained from the second homogenization was labeled as homogenate #2), and the homogenized bacterial solution was collected upon completion. The homogenized bacterial solution was aliquoted into centrifuge tubes, centrifuged at 17,000 rpm and 4 °C for 30 minutes, and the supernatant (labeled as supernatant) was collected. Both the supernatant and the pellet (labeled as pellet) were subjected to electrophoresis detection.

### (3) Purification of recombinant polypeptide TEKB

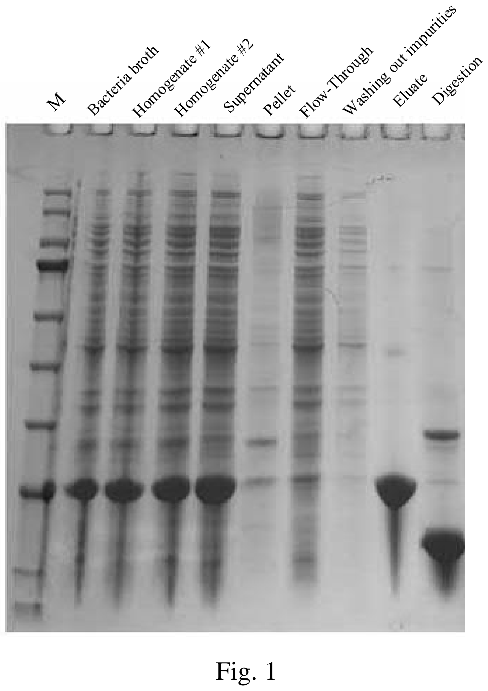

Coarse purification: a. The Ni column material was water-washed for 5 column volumes (CVs); b. The column material was equilibrated with an equilibration solution (200 mM sodium chloride, 25 mM Tris, 20 mM imidazole) for 5 CVs; c. Loading: The centrifuged supernatant was loaded to the column material until the liquid flowed out; d. Washing of impurity proteins: 25 mL of a washing solution (200 mM sodium chloride, 25 mM Tris, 20 mM imidazole) was added until the liquid ran out; e. Collection of the protein of interest: 25 mL of an eluting solution (200 mM sodium chloride, 25 mM Tris, 250 mM imidazole) was added, and the flow-through was collected to obtain the target polypeptide Trx-His-TEKB. If a target polypeptide with the Trx-His tag excised was desired, an appropriate amount of His-tagged TEV protease could be added; after incubation at 16 °C for 2 hours, the polypeptide TEKB (with the Trx-His tag protein removed) was obtained. Fig. 1 showed the electrophoretic detection result of products obtained from various steps in the purification process of polypeptide TEKB, indicating that polypeptide TEKB was present in large quantities in soluble fractions, which facilitated subsequent purification and separation.

In order to further obtain a high-purity polypeptide TEKB, we selected different types of reversed-phase columns (C8BS (Hanbon Science and Technology Co., Ltd.), C18BS (Hanbon Science and Technology Co., Ltd.), Hedera C8 (Hanbon Science and Technology Co., Ltd.), Daisogel SP-100-8-c8-PK (Daisogel)) and the high-purity polypeptide TEKB was obtained using the method described below.
Loading volume: 40 ml
Mobile Phase:

| Time T/min | Flow rate ml/min | A: 0.1% TFA water/% | B: Acetonitrile/% |
|---|---|---|---|
| 0 | 3 | 90 | 10 |
| 60 | 3 | 45 | 55 |

Detection wavelength: 215 nm 270 nm
Volumetric flow rate: 3 ml/min

The purity of the polypeptide was detected using ultra-high performance liquid chromatography (Waters).

The polypeptide TEKB, which exhibited broad-spectrum inhibition of human coronavirus infection, was successfully prepared by the above-mentioned biosynthesis method. The polypeptide TEKB prepared via recombinant expression using an *E. coli* fermentation process featured a simple production procedure and was easy to popularize.

### Example 2: Concentration Detection of Polypeptide TEKB

An appropriate amount of sample was accurately measured, diluted 10-50 times with the eluting solution, and thoroughly stirred evenly with a glass rod. A UV-Vis spectrophotometer was used to measure the absorbance at 280 nm, and the protein concentration was calculated according to the formula: C (mg/ml) = A280 × dilution factor (Note: the absorbance value should be between 0.1 and 1).

After calculation, the concentration of the purified polypeptide TEKB was 7.90 mg/ml; the polypeptide yield from 2 L of fermentation broth was 158 mg, and the protein yield relative to the fermentation broth was as high as 79 mg/L.

### Example 3: Purity Detection of polypeptide TEKB

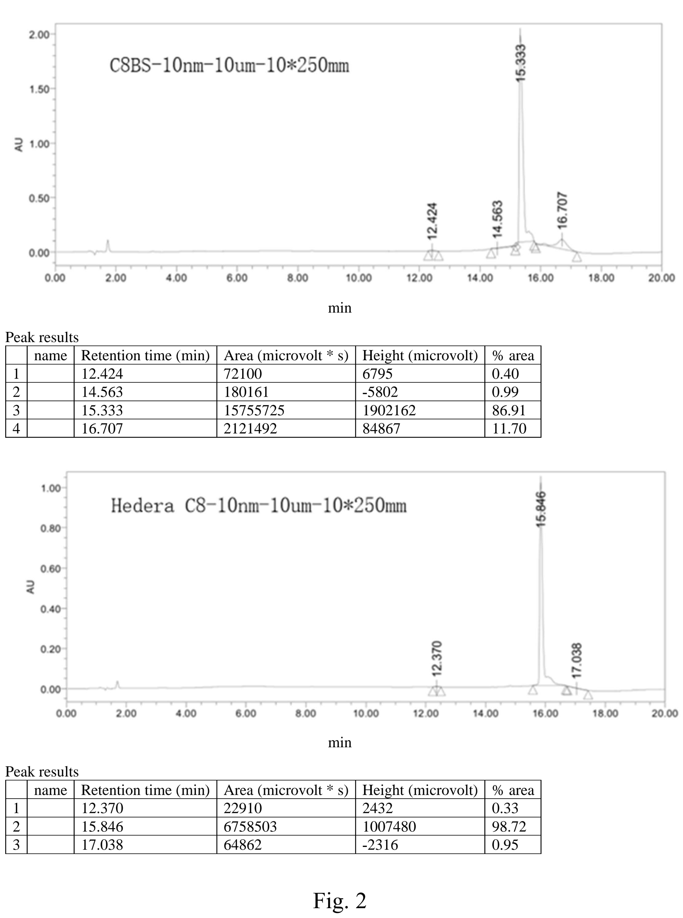

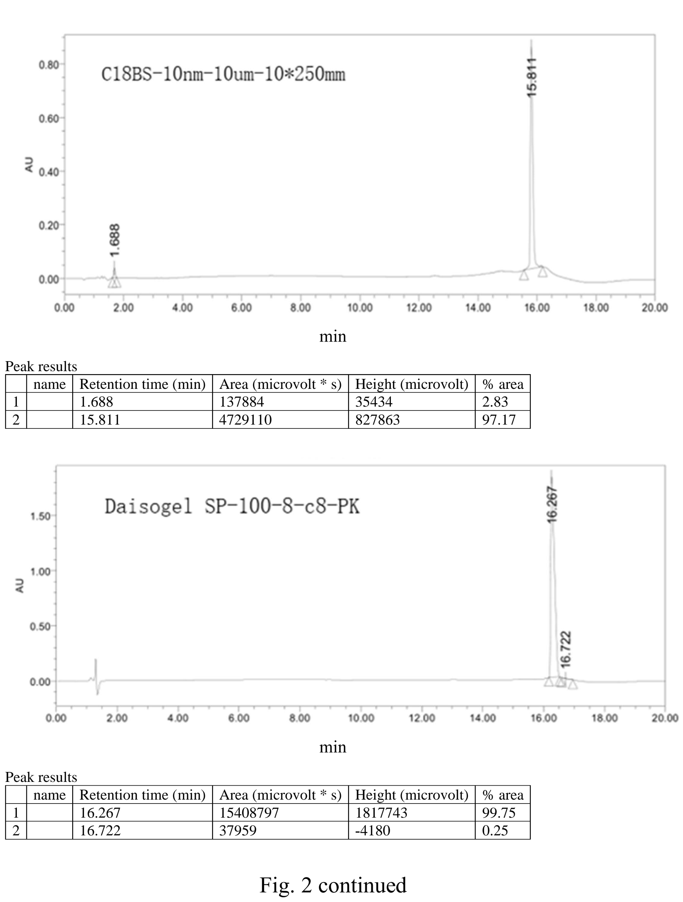

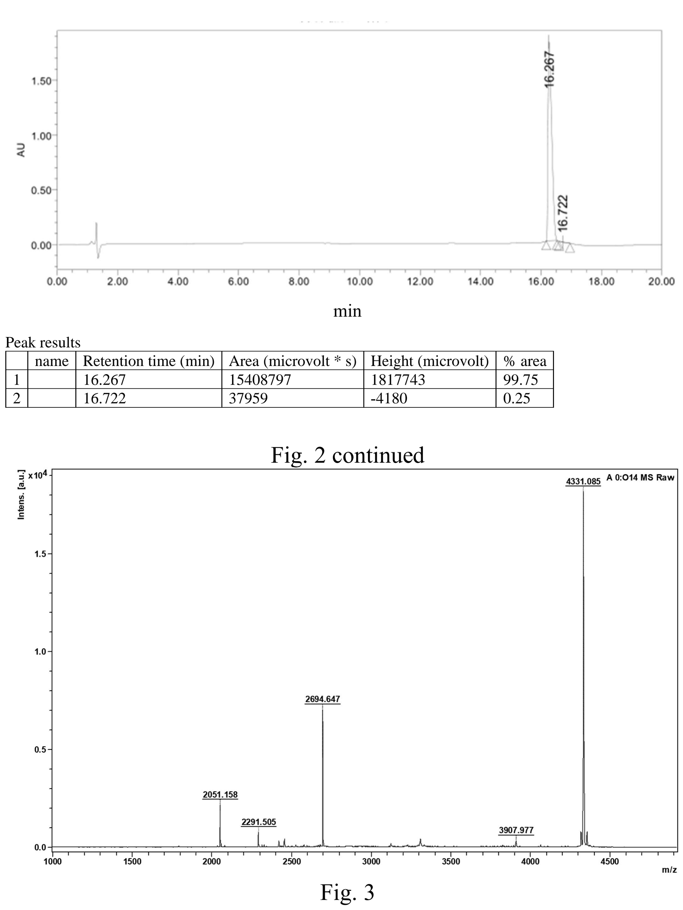

Reversed-phase chromatography (RPC) is a chromatographic mode established based on the hydrophobic interactions between the solute, polar mobile phase, and non-polar stationary phase surface. The partition coefficient of a solute on the reversed-phase medium depends on the hydrophobicity of the solute. Generally, the higher the hydrophobicity, the larger the partition coefficient. The protein test samples were analyzed via RPC in accordance with General Rules of the Pharmacopoeia of the People's Republic of China (2020 Edition), Four Volumes (General Chapter 0512 High Performance Liquid Chromatography), and the purity percentage of the main peak was further calculated using the peak area normalization method. The purities of the polypeptide purified by different types of reversed-phase columns (C8BS-10nm-10µm-10*250mm (Hanbon), C18BS-10nm-10µm-10*250mm (Hanbon), Hedera C8-10nm-10µm-10*250mm (Hanbon), Daisogel SP-100-8-C8-PK (Daisogel)) were 86.91%, 97.17%, 98.72%, and 99.75%, respectively. As shown in Fig. 2, the polypeptide TEKB purified using the Daisogel SP-100-8-C8-PK (Daisogel) column exhibited the highest purity, reaching 99.75%. Unexpectedly, the inventors found that the Daisogel SP-100-8-C8-PK (Daisogel) column was capable of producing high-purity polypeptide TEKB, rendering it suitable for pharmaceutical production.

### Example 4: Mass spectrometric detection of polypeptide TEKB

Since the theoretical molecular weight of the polypeptide TEKB was small and could not be detected or visualized by conventional SDS-PAGE, the purified polypeptide TEKB was subjected to protein molecular weight determination: the sample was desalted using ziptipC18, then mixed with a matrix (CHCA) and spotted onto a plate. Finally, Matrix-Assisted Laser Desorption/Ionization Time-of-Flight Mass Spectrometer MALDI-TOF/TOF Ultraflextreme^{™}, Bruker, Germany was employed for analysis in reflection mode.

The theoretical molecular weight of the polypeptide TEKB was 4331.98 Da; as this could not be detected by conventional SDS-PAGE, mass spectrometry was required for identification. As shown in Fig. 3, the actual molecular weight of the polypeptide TEKB of the present disclosure identified by mass spectrometry was 4331.085 Da, which was consistent with the theoretical molecular weight.

### Example 5: Preparation of pseudoviruses of SARS-CoV-2 and its variants.

### Experimental method:

1. 293T cells were digested 24 hours prior to transfection and plated in a 10 cm tissue culture dish (2 x 10⁶ / dish);
2. The cells were replenished with pre-warmed fresh DMEM medium (containing 10% FBS) 2 hours before transfection;
3. Two 1.5 mL EP tubes were prepared for the transfection procedure. EP tube #1 was added with 500 µL of 0.9% NaCl solution containing 20 µg of pcDNA3.1-plasmid (or pcDNA3.1-SARS-CoV-2 (D614G)-S plasmid, with S protein sequence referenced in EPI_ISL_412912; or pcDNA3.1-BA.2 (EPI_ISL_9022266), BA.2.75 (EPI_ISL_13583747), BA.2.86 (EPI_ISL_18138566), XBB.1.5 (EPI_ISL_16346200), or EG.5-CoV-S (EPI_ISL_18052033)) along with pNL4-3.luc.RE plasmid; EP tube #2 was added with 500 µL of 0.9% NaCl, followed by the addition of 10 µL of the transfection reagent Vigofect, and both EP tubes were allowed to stand for 5 minutes.
4. 500 µL of the solution from EP tube #2 was added dropwise to EP tube #1, mixed homogeneously with a pipette tip during addition, and the resulting mixture was incubated at room temperature for 15 minutes.
5. 1 mL of the above mixed solution was added dropwise and evenly to the culture dish pre-plated with 293T cell;

At 8-10 hours post-transfection, the medium was replaced with 10 mL of fresh DMEM medium containing 10% FBS;

At 48 hours post-transfection, the supernatant containing a pseudovirus was collected;

The supernatant was centrifuged at 4000 rpm for 4 min to remove cell debris, filtered through a 0.45 µm sterile filter, aliquoted, and stored at -80 °C for subsequent use.

### Example 6: Determination of the inhibitory activity of the prokaryote-expressed polypeptide reEK1 against pseudoviruses of SARS-CoV-2 and its variants

### Experimental method:

1. The test polypeptide drugs (EK1 and reEK1) were dissolved in DMEM, and the polypeptide concentrations were determined.
2. A suspension of target Caco2 cells was prepared, which express a receptor for human coronaviruses. The cell concentration was adjusted, and 10⁴ cells were added to each well.
3. The polypeptide drug was serially diluted in a 96-well plate using DMEM medium containing 10% FBS, with 50 µL each well.
4. A specific titer of pseudovirus was added to the plate with drug dilutions at 50 µL / well. The mixture was incubated at room temperature for 30 minutes to allow full interaction between the drug and the virus. Subsequently, 100 µL of the drug-virus mixture was transferred into the target cells from which the supernatant had been removed. After culturing at 37 °C for 12 hours, the medium was replaced with fresh DMEM medium containing 10% FBS.
5. The original culture medium in the 96-well cell plate was discarded, and 40 µL of cell lysis solution (Promega, Cat. E1531) was added to each well. The plate was shaken on a decolorizing shaker for 45 minutes to lyse the cells. Then, 30 µL of cell lysate was transferred from each well to a 96-well ELISA plate, followed by the addition of 30 µL of firefly luciferase substrate (Promega, Cat. E1501) to each well. The ELISA plate was placed in a microplate reader (PerkinElmer) to measure the fluorescence value of each well. The inhibition rate curve was plotted, and the half-maximal inhibitory dose (IC₅₀) of the drug was calculated. Specifically, the viral inhibition rate for each drug-treated well was calculated using the following formula: Inhibition rate = [(virus well fluorescence value - drug-treated well fluorescence value) / (virus well fluorescence value - cell well fluorescence value)] * 100%. The virus well received no polypeptide treatment, the drug-treated well received polypeptide treatment, and the cell well received no virus treatment. The calculated viral inhibition rates for each well were imported into Graphpad Prism software, and the Variable Slope under the Dose-Response-Inhibition mode was selected to generate the inhibition rate curve and determine the IC₅₀ value.

### Results and discussion:

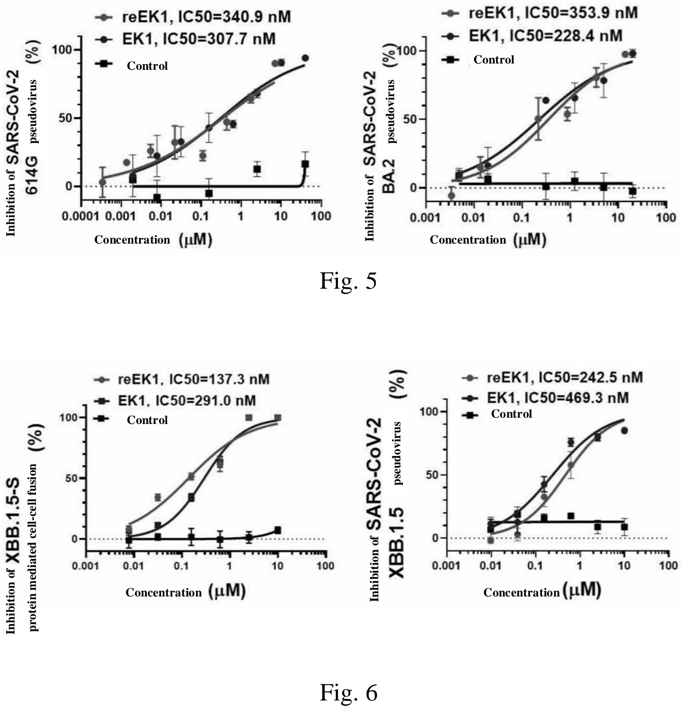

As shown in Figs. 5-8 and Table 2, the prokaryote-expressed polypeptide reEK1 exhibited significant antiviral activity on the pseudoviruses of SARS-CoV-2 and its variants (BA.2, BA.2.75, BA.2.86, XBB.1.5, EG.5), with its IC₅₀ values reaching the nanomolar level. Its antiviral activity was equivalent to that of the chemically synthesized polypeptide EK1, and even showed improvement against certain variants. These results indicate that the reEK1 polypeptide can efficiently and broadly inhibit infection by currently prevalent or future emerging SARS-CoV-2 and its variants. Meanwhile, this also suggests that reEK1 possesses the same broad-spectrum activity as the chemically synthesized EK1 polypeptide and exerts high inhibitory effects against other human coronaviruses as well.

**Table 2. Inhibitory activity of prokaryote-expressed polypeptide reEK against Pseudoviruses of SARS-CoV-2 and variants thereof:**

| **Polypeptide Name** | **Inhibitory activity, IC₅₀ (nM)** | | | | | |
|---|---|---|---|---|---|---|
| | **SARS-CoV-2 (D614G)** | **BA.2** | **BA.2.75** | **BA.2.86** | **XBB.1.5** | **EG.5** |
| **reEK1** | **340.9** | **353.9** | **420.0** | **298.0** | **137.3** | **331.1** |
| **EK1** | **307.7** | **228.4** | **437.9** | **391.7** | **291.0** | **401.2** |

### Example 7: Inhibition of human coronavirus S protein-mediated cell-cell fusion experiment

### Experimental method:

1. 293T cells were cultured for 36-48 hours after transfection with a plasmid encoding human coronavirus S protein and used as effector cells. The S protein-encoding gene was loaded into a pAAV-IRES-GFP vector (purchased from Agilent technologies, USA). Specifically, 293T cells (purchased from the Cell Bank of the Chinese Academy of Sciences) were transfected with the plasmid pAAV-IRES-GFP-BA.2.86 or XBB.1.5-S encoding BA.2.86 or XBB.1.5-S protein to obtain transfected cells, referred to as 293T/S/EGFP cells. 293T cells were transfected with the empty plasmid pAAV-IRES-GFP to obtain 293T/EGFP cells, which were used as negative control cells.
2. 293T/S/EGFP cells and 293T/EGFP cells were digested with 0.02% EDTA, centrifuged, resuspended with fresh DMEM medium containing 10% FBS, and the cell concentration was adjusted to 2 x 10⁵ cells/mL, and then 50 µL was removed and added to various serially diluted test peptide drugs (50 µL; EK1 and reEK1), incubated at 37 °C for 30 min.
3. 100 µL of the cell/drug mixture was added to target cells Caco2 (purchased from the Cell Bank of the Chinese Academy of Sciences) which had been plated on a 96-well plate. The cells were cultured with 5% CO₂ and at 37°C for 2-4 hours, and the fusion of cells was observed and recorded using the green fluorescence channel of a fluorescence microscope..

### Results and discussion:

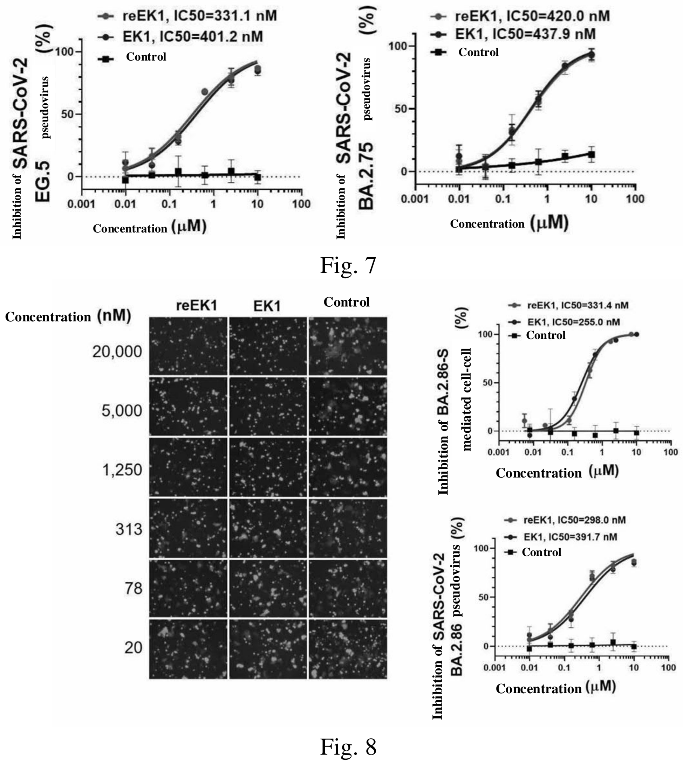

As shown in Figs. 6 and 8, on the cell-cell fusion assays of SARS-CoV-2 and its variants (BA.2.86, XBB.1.5), the prokaryote-expressed polypeptide reEK1 has significant antiviral effect, with an IC50 in the low nanomolar range. Its inhibitory efficacy was comparable to that of the chemically synthesized polypeptide EK1.

### Example 8: In vivo antiviral effect of reEK1 in hACE2 transgenic mouse model

The Omicron variant has led to numerous reported cases of vaccine "breakthrough" infections and "reinfections" recently. The efficacy of reEK1 against Omicron (BA.2) (provided by the Fudan University P3 Laboratory) was evaluated in a Tgtn (CAG-human ACE2-IRES-Luciferase) transgenic mouse model.

### Experimental method:

1. Tgtn (CAG-human ACE2-IRES-Luciferase) transgenic mouse model (purchased from Shanghai Model Organisms Center, Inc.)
   a. Each mouse was challenged with 30,000 pfu (50 µL) of Omicron (BA.2).
   b. Two hours post-challenge, each mouse was administered a dose of 200 µg of reEK1 via nebulization, once daily for 3 consecutive days.
   c. The mice were euthanized on day 7 post-infection, and the viral load and lung pathological changes in the lung tissues of mice in each group were detected.

### Results and analysis:

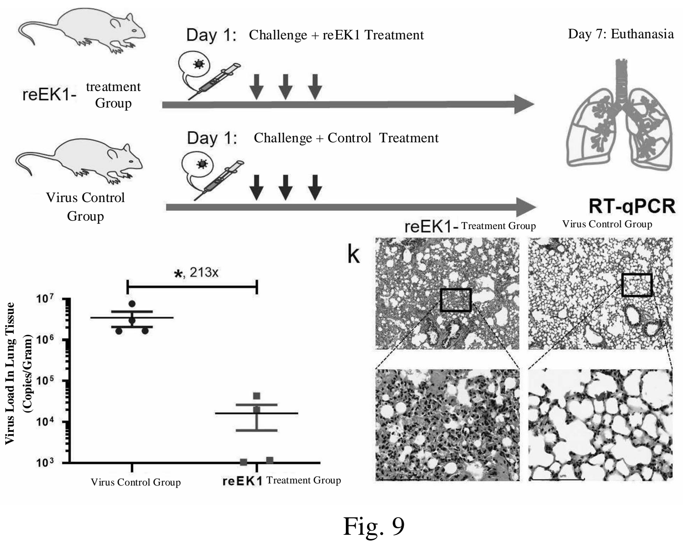

As shown in Fig. 9, the viral load in the lungs of Omicron (BA.2)-infected mice increased significantly at day 7, whereas the viral titer in the lungs of reEK1-treated mice decreased dramatically (by 213-fold). Additionally, the reEK1 treatment group exhibited significant amelioration of lung pathological changes, which further demonstrates that reEK1 exerts an excellent *in vivo* antiviral effect.

### Discussion

In summary, the present disclosure is the first to discover that the prokaryote-recombinantly expressed polypeptide (reEK1) exerts an excellent antiviral activity against SARS-CoV-2-D614G and its variants (BA.2, BA.2.75, BA.2.86, XBB.1.5, EG.5), and also provides effective *in vivo* antiviral protection. This polypeptide thus serves as an excellent candidate drug for the prevention and treatment of currently prevalent SARS-CoV-2 variants as well as newly emerging SARS-CoV-2 variants that may outbreak in the future.

In 2016 and 2019, Lu Lu, Jiang Shibo, Xia Shuai (co-inventors of the present patent) and their research team first disclosed the sequence of the chemically synthesized broad-spectrum antiviral polypeptide EK1 (patent application numbers: CN201610070216.4; CN107022008B), and reported that this chemically synthesized polypeptide exhibits broad-spectrum antiviral effects against SARS-CoV-2 and its variants, SARS-CoV, SARS-like viruses Rs3367-CoV, or W1V1-CoV, MERS-CoV, HCoV-229E, and HCoV-NL63. These findings were published in *Cell Research* (a Nature sub-journal) and *Science Advances* (a Science sub-journal) (References: Xia S, Liu M, Wang C, Xu W, Lan Q, Feng S, Qi F, Bao L, Du L, Liu S, Qin C, Sun F, Shi Z, Zhu Y, Jiang S, Lu L. Inhibition of SARS-CoV-2 (previously 2019-nCoV) infection by a highly potent pan-coronavirus fusion inhibitor targeting its spike protein that harbors a high capacity to mediate membrane fusion. Cell Res. 2020 Apr;30(4):343-355 and Xia, S., Yan, L., Xu, W., Agrawal, A.S., Alsaissi, A., Tseng, C.T.K., Wang, Q., Du, L., Tan, W., Wilson, I.A., Jiang, S., Yang, B., Lu, L. A pan-coronavirus Fusion Inhibitor Targeting the HR1 Domain of Human Coronavirus Spike. Science Advances. 2019 Apr 10;5(4):eaav4580). Prior relevant studies have demonstrated that the antiviral activity of prokaryote-expressed polypeptides is significantly lower than that of chemically synthesized polypeptides with identical sequences (Reference: Zhu J, Xiao G, Xu Y, Yuan F, Zheng C, Liu Y, Yan H, Cole DK, Bell JI, Rao Z, Tien P, Gao GF. Following the rule: formation of the 6-helix bundle of the fusion core from severe acute respiratory syndrome coronavirus spike protein and identification of potent peptide inhibitors. Biochem Biophys Res Commun. 2004 Jun 18;319(1):283-8. doi: 10.1016/j.bbrc.2004.04.141. PMID: 15158473; PMCID: PMC7111185). In the present disclosure, however, the prokaryote-recombinantly expressed reEK1 achieves antiviral activity equivalent to that of the chemically synthesized EK1 against SARS-CoV-2 and its various variants, and even demonstrates enhanced inhibitory effects against certain variants, a result that is unexpected. Meanwhile, these findings also indicate that the prokaryote-recombinantly expressed reEK1 possesses broad-spectrum antiviral activity against SARS-CoV, SARS-like viruses Rs3367-CoV or W1V1-CoV, MERS-CoV, HCoV-229E, and HCoV-NL63 as well.

The examples described above are preferred implementations of the present disclosure; however, the embodiments of the present disclosure are not limited to the aforesaid examples. Any other alterations, modifications, substitutions, combinations, and simplifications made without departing from the spirit and principle of the present disclosure shall be deemed equivalent substitutions and shall fall within the scope of protection of the present disclosure.

## Claims

1. A method of producing a polypeptide of SEQ ID NO: 1 by fermentation, comprising fermenting a host cell under a condition suitable for culturing the host cell to collect the polypeptide in a culture supernatant or the host cell, wherein the host cell is a prokaryote host cell and comprises the polynucleotide of SEQ ID NO: 2.

2. The method of claim 1, wherein the host cell is an *E. coli* cell, preferably *E. coli* BL21 (DE3), and the method comprises one or more of the following steps:
(1) culturing the *E. coli* cell in LB medium to obtain a seed culture;
(2) scaling up the seed culture for an appropriate time, and performing an induction culture;
(3) collecting and lysing the *E. coli* cell, and separating the lysate to obtain a supernatant and a pellet;
(4) loading the supernatant into a column to separate and collect the polypeptide; and
(5) enzymatically digesting the polypeptide to remove a tag linked to the polypeptide.

3. The method of claim 2, wherein the step (1) comprises culturing the *E. coli* cell in LB medium at 35-38 °C, preferably at 37 °C for 6-20 hours, preferably 8-16 hours to obtain the seed culture,
the step (2) comprises adding the seed culture to an expansion medium, preferably LB medium, in a ratio of the expansion medium to the seed culture of 10: 1 to 200: 1, preferably 50: 1 to 150: 1, more preferably 80 to 120: 1 followed by incubation at 35-38 °C, preferably 37 °C for 6-10 hours, preferably 7-8 hours; conducting induction by adding IPTG at a IPTG concentration of 0.2-1 mM, preferably 0.3-8 mM, preferably 0.5 mM, cooling to 10-20 °C, preferably 12-18 °C, preferably 16 °C, conducting induction culture for 6-20 hours, preferably 8-16 hours, and collecting the cells;
the step (3) comprises resuspending the cell in a buffer solution, cooling to ≤ 15 °C, performing a homogenization process, preferably a high-pressure homogenization process, to obtain a homogenate, and then separating the homogenate into the supernatant and the pellet, for example by centrifugation;
the step (4) comprises equilibrating column material with an equilibration solution, loading the supernatant into the column material, washing the column with a washing solution to remove impurities, and eluting the column with an eluting solution to obtain the polypeptide, preferably the column material is a nickel column; and/or
the step (5) comprises incubating the polypeptide with a TEV protease, preferably a TEV protease having a His tag, for example at 10-20 °C for 1-8 hours, such as at 16 °C for 2 hours, to obtain the polypeptide.

4. The method of claim 3, wherein the equilibration solution and/or the washing solution comprises 100-400 mM, such as 200-300 mM sodium chloride, 10-50 mM, such as 20-40 mM Tris and 10-50 mM, such as 20-40 mM imidazole; the eluting solution comprises 100-400 mM, such as 200-300 mM sodium chloride, 10-50 mM, such as 20-40 mM Tris and 100-500 mM, such as 200-400 mM imidazole.

5. The method according to any one of claims 1-4, further comprising the step of subjecting the culture supernatant or lysate or eluate comprising the polypeptide to a reversed-phase column chromatography, preferably using a reversed-phase column packed with a material having the following properties:
(1) the shape of the silica gel particles is spherical; (2) the pore size is 100-1000 angstroms; (3) the particle size is 3-15 µm; and (4) the bonded phase is ODS, C1, C4, C8, or APS; more preferably, the reversed-phase column is a Daisogel SP-100-8-c8-PK type reversed-phase column; and the purification is performed under one or more of the following conditions:
mobile phase: 0-60 min, 90% aqueous solution containing 0.1% TFA and 10% acetonitrile; 60 min, 45% aqueous solution containing 0.1% TFA and 55% acetonitrile; detection wavelength: 215 nm and 270 nm; volumetric flow rate: 3 ml/min.

6. The method of any one of claims 1-5, wherein the host cell comprises a vector comprising the polynucleotide of SEQ ID NO: 2, preferably wherein the 5' end of the polynucleotide is linked to the nucleotide sequence of a TEV protease cleavage site to form an insertion cassette; preferably, the TEV protease cleavage site has the amino acid sequence of ENLYFQ; preferably, the vector comprises one or more of an N-terminal 6x His tag, a thrombin cleavage site, a C-terminal 6x His tag, a TrxA site, an enterokinase cleavage site, and an S tag; preferably, the vector is an expression vector, preferably a pET-32a (+) expression vector; preferably, the insertion cassette is inserted into the vector at Kpn I and Xho I sites.

7. An *in vitro* method of inhibiting a coronavirus comprising applying a polypeptide of SEQ ID NO: 1 or a composition comprising the polypeptide, wherein the polypeptide has no acetyl group at the N-terminal amino acid residue and/or has no amide group at the C-terminal amino acid residue or is not acetylated at the N-terminal amino acid residue and/or not amidated at the C-terminal amino acid residue; or the polypeptide is a polypeptide expressed by a prokaryote host cell or is not a chemically synthesized polypeptide, wherein the coronavirus is selected from the group consisting of SARS-CoV-2, Rs3367-CoV, WIV1-CoV, MERS-CoV and HCoV-229E; preferably, the SARS-CoV-2 is selected from the group consisting of BA.2, BA.2.75, BA.2.86, XBB.1.5 and EG.5 variants; preferably, the polypeptide or the composition comprising the polypeptide is administered to a cell infected with or at risk of being infected with a coronavirus or is contacted with a coronavirus; preferably, the polypeptide is a polypeptide prepared by the method of any one of claims 1-6; preferably, the composition further comprises an agent for inhibiting coronavirus or for treating and/or preventing a disease caused by the coronavirus, preferably the agent is selected from the group consisting of favipiravir, nelfinavir, arbidol, lopinavir, ritonavir, chloroquine phosphate, darunavir, or remdesivir; preferably, the composition comprises a pharmaceutically acceptable carrier.

8. Use of the polypeptide of SEQ ID NO: 1 or a composition comprising the polypeptide in the manufacture of a medicament or a kit for preventing or treating a coronavirus infection or a disease caused by a coronavirus in a subject, wherein the polypeptide has no acetyl group at the N-terminal amino acid residue and/or has no amide group at the C-terminal amino acid residue or is not acetylated at the N-terminal amino acid residue and/or not amidated at the C-terminal amino acid residue; or the polypeptide is a polypeptide expressed by a prokaryote host cell or is not a chemically synthesized polypeptide, wherein the coronavirus is selected from the group consisting of SARS-CoV-2, Rs3367-CoV, WIV1-CoV, MERS-CoV and HCoV-229E; preferably, the SARS-CoV-2 is selected from the group consisting of BA.2, BA.2.75, BA.2.86, XBB.1.5 and EG.5 variants; preferably, the polypeptide is a polypeptide prepared by the method of any one of claims 1-6; preferably, wherein the dosage form of the medicament is a tablet, a capsule, a dropping pill, an aerosol, a pill, a powder, a solution, a suspension, an emulsion, a granule, a liposome, a transdermal agent, a suppository or a lyophilized powder injection; preferably, the composition further comprises an agent for inhibiting coronavirus or for treating and/or preventing a disease caused by coronavirus, wherein preferably the agent is selected from the group consisting of favipiravir, nelfinavir, arbidol, lopinavir, ritonavir, chloroquine phosphate, darunavir, or remdesivir; preferably, the composition comprises a pharmaceutically acceptable carrier; wherein the medicament is preferably administered by injection, including subcutaneous injection, intravenous injection, intramuscular injection and intraperitoneal injection, intracisternal injection or infusion, and the like, cavity administration, such as rectally, vaginally and sublingually, respiratory administration, such as nasally; mucosal administration or topical administration.
